# EUROPEAN PATENT SPECIFICATION

(11) **EP 0 663 830 B1**
(45) Date of publication and mention of the grant of the patent: **17.04.2002**
(21) Application number: 92922217.2
(22) Date of filing: 09.10.1992
(51) Int. Cl.: A61K 31/7072, A61P 11/10, A61P 11/12

(54) **METHOD OF TREATING LUNG DISEASE WITH URIDINE TRIPHOSPHATES**
VERFAHREN ZUR BEHANDLUNG VON LUNGENERKRANKUNGEN MIT URIDIN-TRIPHOSPHATEN
METHODE DE TRAITEMENT DE MALADIE PULMONAIRE A L'AIDE D'URIDINE-TRIPHOSPHATES

(43) Date of publication of application: 26.07.1995
(73) Proprietor: THE UNIVERSITY OF NORTH CAROLINA AT CHAPEL HILL, Chapel Hill, NC 27599-4100 (US)
(72) Inventor: BOUCHER, Richard C., Jr., Chapel Hill, NC 27514 (US)
(74) Representative: Horner, Martin Grenville
(86) International application number: PCT/US92/08620
(87) International publication number: WO 94/08593

(56) References cited:
- WO-A-92/11016
- US-A- 4 758 553
- FDC REPORTS: THE BLUE SHEET , vol. 34, no. 35, 28 October 1991 pages 4-5, 'Aerosolized amiloride plus atp or utp therapy stimulates lung secretion in cystic fibrosis patients'
- NEW ENGL.J.MED., vol. 325, no. 8, 22 October 1991 pages 533-538, 'Activation by extracellular nucleotides of chloride secretion in the airway epithelia of patients with cystic fibrosis'
- BR.J.PHARMACOL., vol. 103, no. 3, July 1991 pages 1649-56, 'Regulation of transepithelial ion transport and intracellular calcium by extracellular ATP in human normal and cystic fibrosis airway epithelium'
- British Journal of Pharmacology, Volume 89, issued 1986, W.R. RICE et al., "P2 - Purinoceptors Regulate Surfactant Secretion from Rat Isolated Alvedar Type II Cells", pages 485-489, entire document.
- Annals of the New York Academy of Science, Volume 603, issued 1990, N. CUSACK et al., "Subtypes of P2 - Purinoceptors", pages 172-181, see pages 174 and 175.

## Description

This invention was made with Government support under Grants HL34322 and HL42384 from the National Institutes of Health. The Government may have certain rights to this invention.

### Field of the Invention

This invention relates to a medicament useful for removing retained mucus secretions from the lungs of a patient comprising certain uridine triphosphates.

### Background of the Invention

Extracellular adenosine triphosphate has been shown to regulate a variety of biological processes including non-vascular smooth muscle contraction (M. Maguire and D. Satchell, *J. Pharmacol. Exp. Ther.* **211**, 626-631 (1979); C. Brown and G. Burnstock, *Eur. J. Pharmacol.* **69,** 81-86 (1981)) and vascular tone (G. Burnstock and C. Kennedy, *Circ. Res.* **58,** 319-330 (1986); D. Haeussinger et al., *Eur. J. Biochem.* **167,** 65-71 (1987)), platelet aggregation (G. Born and M. Kratzer, *J. Physiol. (Lond.)* **354,** 419-429 (1984)), neurotransmission (G. Burnstock, Nature **229,** 282-283 (1971); G. Burnstock and P. Sneddon, *Clin. Sci.* **68** (Suppl. 10), 89s-92s (1985)), and cellular ion transport (G. Burgess et al., *Nature* **279,** 554-546 (1979); D. Gallacher, *Nature* **296,** 83-86 (1982)) and secretory activities (J. Chapal and M-M. Loubatieres-Mariani, *Br. J. Pharmacol.* **73,** 105-110 (1981): J. Pearson et al., Biochem. J. **214,** 273-276 (1983)). These effects are mediated by specific purinergic receptors which response to ATP or other nucleotides present in the extracellular millieu (J. Gordon, *Biochem. J.* **233,** 309-319 (1986)).

Purinoceptors have been functionally identified in rat pulmonary epithelia in studies of regulation of alveolar Type II surfactant phospholipid secretion (W. Rice and F. Singleton, *Br. J. Pharmacol.* **89,** 485-491 (1986)). To our knowledge these receptors have not been reported in human airway epithelial cells. Because ion transport appears to be regulated by purinergic receptor stimulation in other epithelia (Burgess et al., *supra* (1979); Gallacher, *supra* (1982)), several features of the effect of extracellular nucleotides on the ion transport activities of human airway epithelium were investigated by Mason et al., (*Br. J. Pharmacol.,* **103,** 1649-1656 (1991)). In this study, the application of nucleotides to the apical and basolateral membranes of human epithelia was found to induce a concentration-dependent modulation in the ion transport rates across the membranes.

Purinergic receptor regulation of ion transport might have potential therapeutic benefits in lung diseases characterized by abnormalities in epithelial ion transport, e.g., cystic fibrosis. In cystic fibrosis the airway epithelial dysfunction is expressed in part by defective regulation of Cl⁻ ion transport by secretagogues that regulate the apical cell membrane Cl⁻ channel by cAMP-dependent or protein kinase C dependent mechanisms (R. Boucher et al., *J. Clin. Invest.* **78,** 1245-1252 (1986); R. Boucher et al., *J. Clin. Invest.* **84,** 1424-1431 (1989); J. Riordan et al., *Science* **245,** 1066-1073 (1989); J. Rommens et al., *Science* **245,** 1059-1065 (1989)). Induction of Cl secretion of CF airway epithelia *in vivo* might help liquify the relatively dehydrated, thick airway surface liquid that characterizes this disease. We therefore tested whether nucleotides would bypass regulatory defects in CF airway epithelia and induce Cl⁻ secretion at rates similar to those of normal airway cells. The present invention is based upon this investigation.

### Summary of the Invention

According to a first aspect of the present invention, there is provided a pharmaceutical formulation comprising a compound of Formula I below, or a pharmaceutically acceptable salt thereof (hereinafter referred to as the "active compound"), in an amount effective to hydrate lung mucous secretion, in a pharmaceutically acceptable carrier: 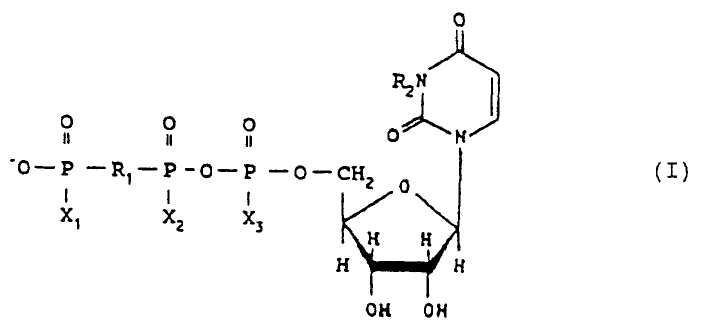 wherein:
X₁ is S⁻;
X₂, and X₃ are each independently selected from the group consisting of O⁻ and S⁻. Preferably, X₂ and X₃ are O⁻.
R₁ is selected from the group consisting of O, imido, methylene and dihalomethylene (e.g., dichloromethylene, difluoromethylene). Preferably, R₁ is oxygen.
R₂ is H.

A particularly preferred compound of Formula (I) above is urdine 5'-*O*-(3-thiotriphosphate) (UTPγS).

The pharmaceutical formulation may further contain amiloride in an amount effective to inhibit the reabsorption of water from lung mucous secretions.

According to a second aspect of the present invention there is provided a pharmaceutical formulation comprising a compound of Formula I, or a pharmaceutically acceptable salt thereof, in an amount effective to hydrate lung mucous secretions, in a pharmaceutically acceptable carrier wherein:
X₁, X₂, and X₃ are each independently selected from the group consisting of O⁻ and S⁻. Preferably X₂ and X₃ are OH.
R₁ is selected from the group consisting of O, imido, methylene, and dihalomethylene. Preferably R₁ is oxygen.
R₂ is H;
with said compound of Formula I subject to the proviso that uridine 5'-triphosphate is excluded therefrom.

According to a third aspect of the present invention there is provided a pharmaceutical formulation comprising amiloride in an amount effective to inhibit the reabsorption of water from lung mucus secretions and a compound of Formula I, or a pharmaceutically acceptable salt thereof, in an aerosolizable form to be delivered to the lungs of a subject in an amount effective to hydrate lung mucous secretions, in a pharmaceutically acceptable carrier wherein:
X₁, X₂, and X₃ are each independently selected from the group consisting of O⁻ and S⁻. Preferably X₂ and X₃ are O⁻.
R₁ is selected from the group consisting of O, imido, methylenem and dihalomethylene. Preferably R₁ is oxygen.
R₂ is H;
with said compound of Formula I subject to the proviso that uridine 5'-triphosphate is excluded therefrom.

The carrier of the pharmaceutical formulation may be selected from the group consisting of solid carriers and liquid carriers.

The pharmaceutical formulation may further comprise a composition wherein the compound of Formula I may be in the form of respirable particles ranging in size from 1 to 10 microns.

### Brief Description of the Drawings

**Figure 1** shows the log concentration-effect curves (percent change in I_{sc} from basal levels) of nucleotides applied to the basolateral surface of normal human nasal epithelium. s.e. of each data point is ≤ 15% of the normalized maximum response.

**Figure 2** shows the log concentration-effect curves (percent change in I_{sc} from basal levels) of nucleotides applied to the apical surface of human nasal epithelium pretreated with amiloride (10⁻⁴ M). s.e. of each data point is ≤ 13 % of the normalized maximum response.

**Figure 3** shows the log concentration-effect relationships of purinergic and pyrimidinergic compounds on [Ca²⁺]ᵢ (mean change in [Ca²⁺]₁ over basal levels) . **(A)** comparison of agonists with bind P_{2X}, P_{2Y} or UTP sensitive receptors; **(B)** Comparison of other purine agonists with response stimulated by ATP; **(C)** Comparison of UTP with other pyrimidine agonists. s.e. of each data point is ≤ 12% of the normalized maximum response.

**Figure 4** shows the representative bioelectric tracings of effect on I_{sc} of extracellular ATP or UTP applied to the apical surface of amiloride pretreated CF human nasal epithelium. **(A)** Cl⁻ secretion in response to ATP; (B) I_{sc} response to ATP of opposite polarity; (C) Cl⁻ secretory response to UTP.

**Figure 5** shows the log concentration-effect curves for changes in I_{sc} from basal levels when ATP or UTP are applied to the apical surface of amiloride-pretreated CF tissues. s.e. of each data point is ≤ 13 % of the normalized maximum response.

**Figure 6** shows the log concentration-effect relationships of the effect of ATP and UTP on [Ca²⁺]ᵢ (change from basal levels) in single CF nasal epithelial cells. s.e. of each data point is ≤ 8 % of the normalized maximum response.

### Detailed Description of the Invention

The medicament of the present invention may be used to hydrate mucous secretions in the lungs of a subject in need of such treatment for any reason, including (but not limited to) retained secretions arising from airway diseases such as cystic fibrosis, chronic bronchitis, asthma, and bronchiectasis. Hydration of the mucous secretions causes allows them to be more easily transported from the lungs via mucociliary action, and hence facilitates the removal of retained mucous secretions.

The present invention is concerned primarily with human subjects, but may also be employed for other mammalian subjects, such as dogs and cats, for veterinary purposes.

Compounds illustrative of the compounds of Formula (I) above include: (a) uridine 5'-triphosphate (UTP) and (b) uridine 5'-*O*-(3-thiotriphosphate) (UTPγS). These compounds are known or may be made in accordance with known procedures, or variations thereof which will be apparent to those skilled in the art. *See generally* N. Cusack and S. Hourani, *Annals N.Y. Acad. Sci.* **603,** 172-181 (G. Dubyak and J. Fedan Eds. 1990)(titled "Biological Actions of Extracellular ATP"). For example, UTP may be made in the manner described in Kenner et al., *J. Chem. Soc.* **1954,** 2288; or Hall and Khorana, *J. Chem. Soc.* **76,** 5056 (1954). See Merck Index, Monograph No. 9795 (11th Ed. 1989). UTPγS may be made in the manner described in G. Goody and F. Eckstein, *J. Am. Chem. Soc.* **93**, 6252 (1971).

For simplicity, Formula I herein illustrates uridine triphosphate active compounds in the naturally occuring D configuration, but the present invention also encompasses compounds in the L configuration, and mixtures of compounds in the D and L configurations, unless specified otherwise. The naturally occuring D configuration is preferred.

The active compounds disclosed herein may be administered to the lungs of a patient by any suitable means, but are preferably administered by administering an aerosol suspension of respirable particles comprised of the active compound, which the subject inhales. The respirable particles may be liquid or solid. The particles may optionally contain other therapeutic ingredients such as amiloride, with amiloride included in an amount effective to inhibit the reabsorption of water from airway mucous secretions, as described in U.S. Patent No. 4,501,729). The term "amiloride" as used herein, includes the pharmaceutically acceptable salts thereof, such as (but not limited to) amiloride hydrochloride. The quantity of amiloride included may be an amount sufficient to achieve dissolved concentrations of amiloride on the airway surfaces of the subject of from 10⁻⁷ to 10⁻³ Moles/liter, and more preferably from 10⁻⁶ to 10⁻⁴ Moles/liter.

The active compounds disclosed herein can be prepared in the form of their pharmaceutically acceptable salts. Pharmaceutically acceptable salts are salts that retain the desired biological activity of the parent compound and do not impart undesired toxicological effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (b) salts formed from elemental anions such as chlorine, bromine, and iodine.

Particles comprised of active compound for practicing the present invention should include particles of respirable size: that is, particles of a size sufficiently small to pass through the mouth and larynx upon inhalation and into the bronchi and alveoli of the lungs. In general, particles ranging from 1 to 10 microns in size (more particularly, less than 5 microns in size) are respirable. Particles of non-respirable size which are included in the aerosol tend to deposit in the throat and be swallowed, and the quantity of non-respirable particles in the aerosol is preferably minimized.

Liquid pharmaceutical compositions of active compound for producing an aerosol may be prepared by combining the active compound with a suitable vehicle, such as sterile pyrogen free water. Other therapeutic compounds such as amiloride may optionally be included.

Solid particulate compositions containing respirable dry particles of micronized active compound may be prepared by grinding dry active compound with a mortar and pestle, and then passing the micronized composition through a 400 mesh screen to break up or separate out large agglomerates. A solid particulate composition comprised of the active compound may optionally contain a dispersant which serves to facilitate the formation of an aerosol. A suitable dispersant is lactose, which may be blended with the active compound in any suitable ratio (e.g., a 1 to 1 ratio by weight). Again, other therapeutic compounds such as amiloride may also be included.

The dosage of active compound will vary depending on the condition being treated and the state of the subject, but generally may be an amount sufficient to achieve dissolved concentrations of active compound on the airway surfaces of the subject of from 10⁻⁷ to 10⁻³ Moles/liter, and more preferably from 10⁻⁶ to 3 x 10⁻⁴ Moles/liter. Depending upon the solubility of the particular formulation of active compound administered, the daily dose may be divided among one or several unit dose administrations.

Aerosols of liquid particles comprising the active compound may be produced by any suitable means, such as with a pressure-driven aerosol nebulizer or an ultrasonic nebulizer. See U.S. Patent No. 4,501,729

Aerosols of solid particles comprising the active compound may likewise be produced with any solid particulate medicament aerosol generator. Aerosol generators for administering solid particulate medicaments to a subject produce particles which are respirable, as explained above, and generate a volume of aerosol containing a predetermined metered dose of a medicament at a rate suitable for human administration. One illustrative type of solid particulate aerosol generator comprises a chamber having a rotor mounted therein, which rotor carries a gelatin capsule containing a metered dose of dry particle medicament. In use the capsule is pierced, a patient inhales through the chamber, and the rotor is caused to spin at a speed sufficient to dispense the medicament to thereby form an aerosol of dry particles. A second type of illustrative aerosol generator comprises a pressurized canister containing dry particle medicament in a propellant. The propellant is discharged through a metering valve configured to dispense a metered dose of the dry particle medicament into the atmosphere. The propellant evaporates, leaving an aerosol of dry particle medicament.

The aerosol, whether formed from solid or liquid particles, may be produced by the aerosol generator at a rate of from 10 to 150 liters per minute, more preferably from 30 to 150 liters per minute, and most preferably 60 liters per minute. Aerosols containing greater amounts of medicament may be administered more rapidly.

The present invention is explained in greater detail in the Examples which follow. These examples are intended as illustrative of the invention, ATP, UTP, ATPγS, CTP, GTP, ITP, adenylyl-imidodiphosphate (AMPPNP), β,γ-methylene ATP (β,γ-MeATP), ADPβS, ADP, AMP, UDP, 5-BrUTP, UMP, ATPαS and dipyridamole were obtained from Boehringer Mannheim Biochemicals (Indianapolis, IN). α,β-methylene ATP (α,β-Me ATP) and amiloride were obtained from Sigma Chemicals (St. Louis, MO). 2-methylthio ATP (2MeSATP) was purchased from Research Biochemicals Inc.(Natick, MA). Bioelectric properties were measured with confluent monolayers bathed by Ham's F-12 culture solution without hormone supplements (Gibco, Grand Island, NY). The acetoxymethylester of Fura-2 (Fura-2/AM) and the pentapotassium salt Fura-2 were purchased from Molecular Probes (Eugene, OR). To obtain external calibration standards for the dye, the pentapotassium salt Fura-2 was used at 15 µM in solution with 150 mM KCl, 20 mM NaCl, 10 mM 4-(2-hydroxyethyl)-1-piperazine- ethanesulfonic acid (HEPES), and either 5 mM CaCl₂ or 2 mM EGTA. For intracellular calcium studies NaCl Ringer solution at 25°C was employed, containing the following (in millimolar): 150 NaCl, 5 KCl, 5 D-glucose, 10 HEPES, 2 CaCl₂, 2 MgCl₂, adjusted to pH 7.4. For studies using solutions free of calcium, 2 mM EGTA replaced the CaCl₂.

### EXAMPLE 1

### Cell Culture

Freshly excised human nasal epithelium from normal or cystic fibrosis subjects was grown in primary culture in F-12, hormone-supplemented medium in accordance with known procedures. *See* R. Wu et al., Am. Rev. Respir. Dis. 132, 311-320 (1985). For [Ca²⁺]ᵢ measurements, cultures were grown on non-fluorescent vitrogen substrates on glass coverslips. Cultures for electrophysiologic studies were grown to confluence on permeable collagen matrix supports (CMS), allowing addition of agonists to the basolateral or apical surface of the epithelial sheet.

### EXAMPLE 2

### Bioelectric Studies

Confluent monolayers of primary human nasal epithelium were mounted in modified Ussing chambers in accordance with known procedures. *See, e.g.,* R. Boucher et al., *J. Physiol. (Lond.)* **405,** 77-103 (1988); M. Knowles et al., Science **221,** 1067-1070 (1983). The studies described below were performed under short circuit current conditions at 37°C, and in some studies, amiloride was added to the apical bath (10⁻⁴ M) to block transepithelial sodium transport. Changes in transepithelial potential difference (Vₜ), resistance (Rₜ) and short circuit current (I_{sc}) were measured in response to addition of various nucleotides. Each cultured preparation was exposed to only one concentration of an agonist on either the basolateral or apical surface to avoid the tachyphylaxis observed in preliminary cumulative dose response studies. To construct concentration-effect relationships of responses to nucleotides, it was assumed that the same maximum response to an agonist could be induced from each tissue culture preparation from the same individual.

### EXAMPLE 3

### Measurements of Intracellular Calcium

Primary human nasal epithelial cells grown to confluence on vitrogen coated coverslips were loaded with a final concentration of 3 µM Fura-2/AM at 37°C for 30 minutes. The cells were then washed in NaCl Ringer and mounted in a chamber for measurements of fluorescence. To reduce the rate of leakage of Fura-2 from the cell into the extracellular space and avoid time-dependent compartmentalization of the probe, all measurements of [Ca²⁺]ᵢ were performed at 25°C. At this temperature, no vesicular bright spots indicative of compartmentalization of the probe were observed.

Measurements of [Ca²⁺]ᵢ in single human nasal epithelial cells were obtained with a modular microspectrofluorimeter (SPEX Industries, Inc., Edison, NJ) attached to a Zeiss Axiovert IM 35 microscope. The system was equipped with a xenon lamp, beam splitter, two monochromators and a rotating chopper mirror that permitted excitation of cell fluorescence at alternating wavelengths of 340 and 380 nm (emission ≥ 450 nm). The fluorescent signal from a single cell was measured with a photometer equipped with a pinhole (spot diameter of 3-5 µm) that excluded signals from adjacent cells.

After agonist was added, the fluorescent signal was quenched by a NaCl ringer solution containing 1.5 X 10⁻⁴ M digitonin and 10⁻³ M MnCl₂. The remaining signal at each excitation wavelength, equivalent to the background fluorescence in non-loaded cells, was subtracted from data from Fura-2/AM loaded cells before the ratio (340nm/380nm) was taken. The 340nm/380nm ratio was converted to an actual [Ca²⁺]ᵢ measurement by using the external calibration standards and the formula derived by G. Grynkiewicz et al., *J. Biol. Chem.* **260**, 3440-3450 (1985), used with dual wavelength measurements: [Ca²⁺]ᵢ=K [(Rₓ-Rₒ)/(Rₛ-Rₓ)], with Rₒ and Rₛ representing the ratios at 0 Ca²⁺ and saturating Ca²⁺, respectively. Rₓ represents the experimental ratio. K is K_{d}(Fₒ/Fₛ), with K_{d}=1.57 X 10⁻⁷ M at 25°C as the effective dissociation constant for Fura-2, and Fₒ and Fₛ represent the fluorescence intensities at 380 nm with zero and saturating Ca²⁺, respectively.

### EXAMPLE 4

### Normal Human Nasal Epithelium - Ion Transport

The effects of extracellular ATP on normal human nasal epithelium were investigated employing bioelectric measurements of ion transport activity when the nucleotide was applied to either the apical or basolateral membrane. ATP rapidly stimulated an increase in I_{sc} when applied to either surface (data not shown). In general, the change in I_{sc} induced by application of ATP to the apical or the basolateral side returned to baseline or below within 5 minutes after addition of agonist. Oscillations in I_{sc} following ATP were frequently observed. Apical pretreatment with amiloride (10⁻⁴ M) removes active Na⁺ absorption as a component of the I_{sc} so that the residual I_{sc} reflects a Cl⁻ secretory current (R. Boucher et al., J. Clin. Invest. 78, 1245-1252 (1986); N. Willumsen et al., Am. J. Physiol. 256, C1033-C1044 (1989)).

A typical Cl⁻ secretory response of human nasal epithelium was found when ATP is applied to amiloride-pretreated tissues (data not shown). Following the initial peak, the ATP induced increase in I_{sc} after basolateral addition to amiloride-pretreated tissues returned to baseline within 5 minutes. In contrast, most tissues treated with ATP on the apical surface following amiloride pretreatment exhibited prolonged (> 10 minutes) increases in I_{sc} above baseline levels.

A concentration-effect relationship was found when ATP was applied to normal human nasal epithelium under basal conditions (data not shown). Comparisons were made between responses of tissues from different donors based on the peak change in I_{sc} following ATP application. The curve describes the mean peak change in I_{sc} in response to log increasing concentrations of ATP applied to the apical or basolateral surface. The effectiveness of the nucleotide is approximately equal when applied to the apical or basolateral surface between 10⁻⁷ and 10⁻⁴ M. A large increase in I_{sc} is seen with 10⁻³ M ATP applied to the basolateral membrane that is not seen with apical application.

Concentration-effect relationships were also found for ATP applied to the apical or basolateral membrane of amiloride-pretreated tissues (data not shown). Again, the nucleotide's effect on ion transport was examined as the mean peak change in I_{sc} after ATP application. The change in ion transport induced by ATP in amiloride-pretreated tissues is routinely smaller than that observed in tissues in the basal state. The potency and effectiveness of ATP in amiloride-treated tissues are similar whether applied to the apical or basolateral membrane and the log concentration-effect curves are sigmoidal in character, with EC₅₀ values of approximately 1-2 X 10⁻⁵ M.

The purinergic receptor subtype(s) linked to regulation of ion transport of human nasal epithelium were characterized by obtaining concentration-effect relationships for a variety of purine and pyrimidine agonists in preparations derived from normal and CF patients. We characterized receptor subtype(s) on the basolateral surface by measuring nucleotide effect on basal Na⁺ transport rates. Because therapies designed to induce Cl⁻ secretion might best be delivered by the aerosol route, receptor subtype characterization on the apical barrier was performed in the presence of amiloride. The effect of extracellular nucleotides on ion transport is reported as the percent change in I_{sc} from control values when applied to the basolateral or apical surface of the culture. Basal pre-agonist currents were similar for tissues in each concentration group.

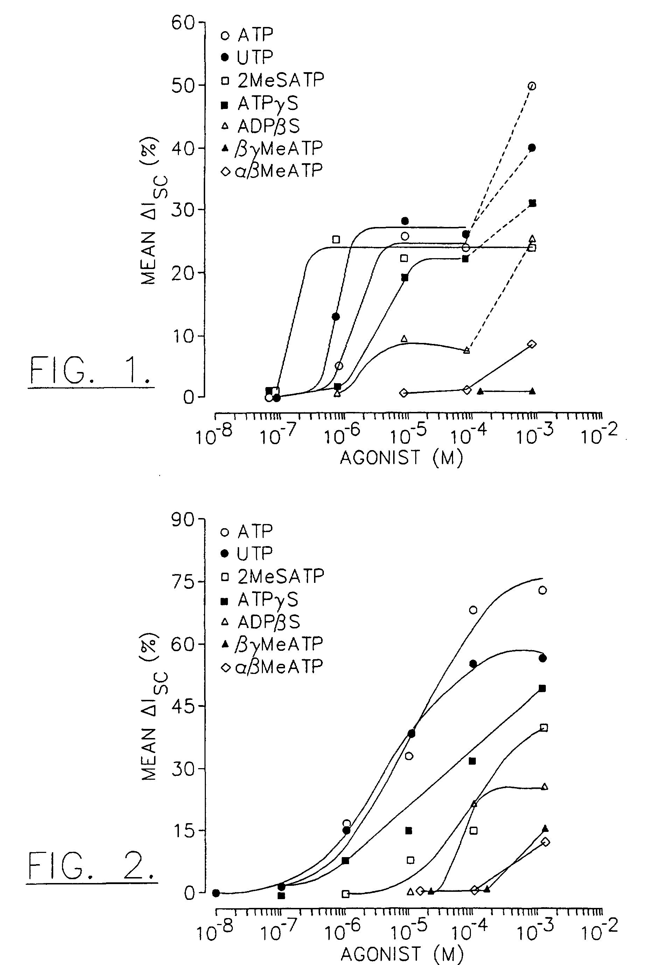

Under the culture conditions employed in these studies, the P₁ receptor agonist adenosine, following preincubation of tissues with dipyridamole [10⁻⁶ M] to block adenosine uptake, induced only small and variable changes in I_{sc} compared to ATP (compare with Figures 1 and 2, below). Addition of adenosine to the apical surface of amiloride-pretreated human nasal epithelium at 10⁻⁵ M (n=7) or 10⁻⁴ M (n=8) induced an increase of 10±7 or 10±6 percent, respectively, whereas addition of the same doses to the basolateral barrier (n=8, each dose) raised I_{sc} by less than 5 percent. These findings suggest that the activation of P₁ receptors contributes little to the measured effects of extracellularly applied ATP on ion transport. Therefore, we focused on agonists that interact with P₂ receptors in the regulation of ion transport and [Ca²⁺]ᵢ mobilization in human nasal epithelium.

**Figure 1** illustrates the concentration-effect relationships of agonists applied to the basolateral surface of airway epithelium. In Figure 1, mean basal I_{sc} in each set of experiments were (in µA cm⁻²): ATP (O), 69±5, range 55-80, n=5 (n=3-11 at each agonist concentration); UTP (●), 66±9, range 21-158, n=9 (n=3-23); 2MeSATP (□), 48±6, range 35-65, n=6 (n=3-17); ATPγS (■), 47±8, range 28-72, n=3 (n=3); ADPβS (Δ), 49±6, range 36-65, n=3 (n=3); αβMeATP (◇), 51±12, range 28-66, n=3 (n=3); βγMeATP (▲), 79±21, range 59-100, n=3 (n=3). Compared to the concentration-effect curve of ATP, agonists that stimulate P₂ₓ receptors (αβMeATP and βγMeATP) induced little change in ion transport rates. The observed rank order of potency for agonists that significantly increased I_{sc} was 2MeSATP > UTP ≥ ATP > ATPγS > ADP > ADPβS. At concentrations of ATP, UTP, ATPγS or ADPβS between 10⁻⁷ and 10⁻⁴ M, the relationship between log agonist concentration and observed responses was a curve of sigmoidal character. The concentration-effect curve of these agonists was biphasic in character when the effect on I_{sc} of 10⁻³ M drug was considered.

The concentration-effect relationships for nucleotides added to the apical surface of amiloride-pretreated tissues are shown in **Figure 2**. In Figure 2, mean post-amiloride I_{sc} in each set of experiments were (in µA cm⁻²): ATP (○), 13±1, range 9-16, n=8 (n=3-11 at each agonist concentration); UTP (●), 12±1, range 11-15, n=5 (n=3-17); ATPγS (■), 12±1, range 8-15, n=3 (n=3); 2MeSATP (□), 16±2, range 12-21, n=3 (n=3-7); ADPβS (Δ), 15±1, range 13-16, n=3 (n=3); βγMeATP (▲), 14±0, range 8-19, n=3 (n=3); αβMeATP (◇), 11±0, range 7-14, n=3 (n=3). Compounds reported to be effective P₂ₓ receptor agonists stimulated little change in ion transport by airway epithelium. Those reported to be effective P_{2y} or UTP sensitive receptor agonists stimulated Cl⁻ secretion with the following rank order of potency: ATP ≥ UTP > ATPγS > ADP > 2MeSATP > ADPβS.

### EXAMPLE 5

### Normal Human Nasal Epithelium - Intracellular Calcium

Based on studies in other epithelia indicating that regulation of [Ca²⁺]ᵢ by purinergic receptors initiates changes in ion transport rates (G. Kimmich and J. Randles, Am. *J. Physiol.* **243,** C116-C123 (1982)), we asked whether ATP regulated [Ca²⁺]ᵢ in single human nasal epithelial cells using intracellular Ca²⁺ sensitive fluorescent dye. Extracellular application of ATP induced an immediate increase in [Ca²⁺]ᵢ levels that decreased over 1 to 2 minutes to a prolonged plateau (data not shown). Exposure to ATP in Ca²⁺-free medium resulted in an initial sharp increase in [Ca²⁺]ᵢ which returned to baseline over a two minute period with no plateau phase observed (data not shown). Return of the cells to a Ca²⁺-containing solution resulted in restoration of the plateau phase in the Ca²⁺ response to ATP.

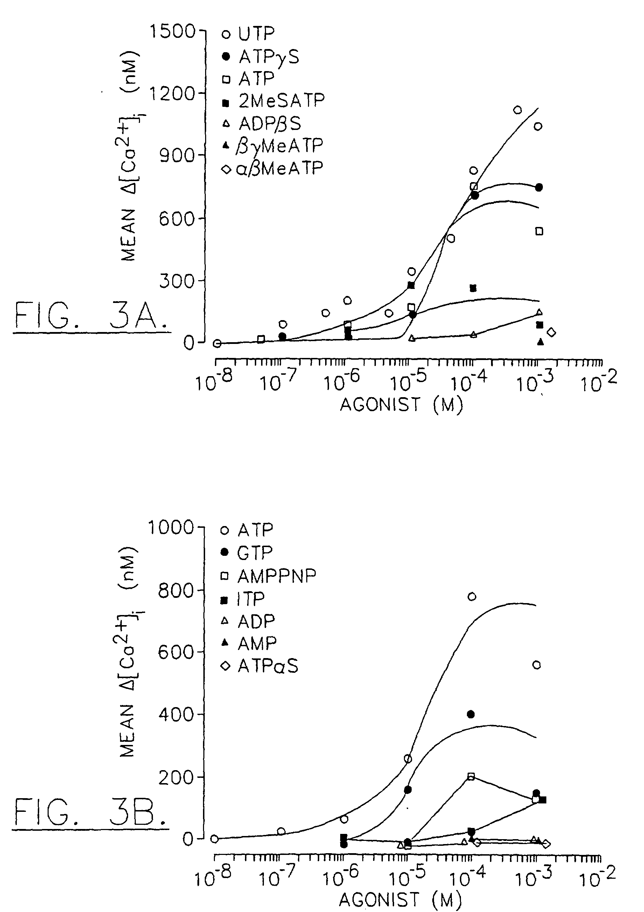

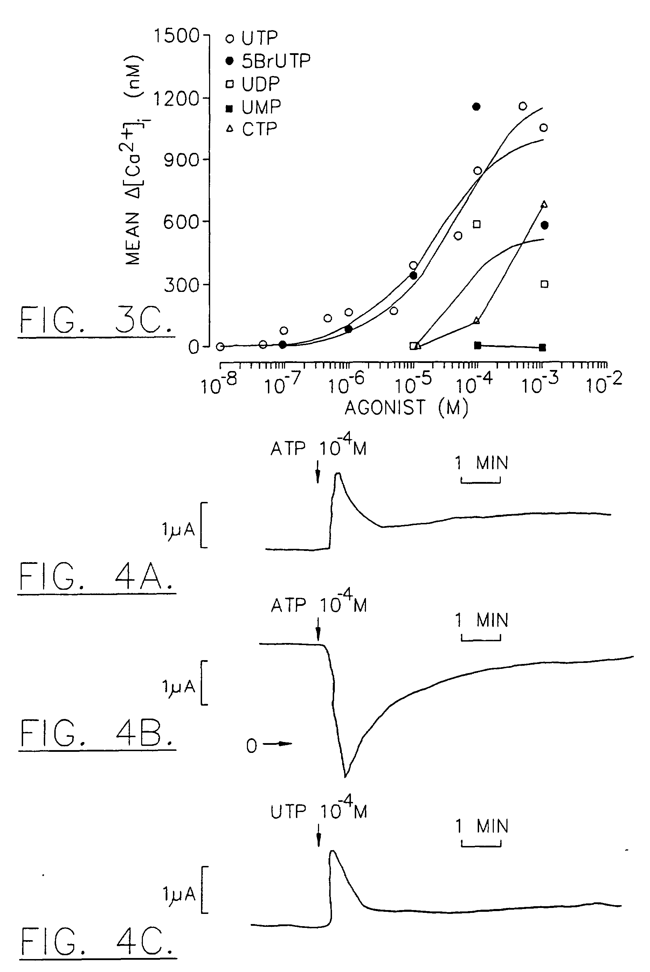

To investigate whether changes in [Ca²⁺]ᵢ might be related to regulation of ion transport, receptor characterization was performed by measuring changes in [Ca²⁺]ᵢ in response to a number of nucleotide drugs. Concentration-effect curves for agonists active at P₂ₓ, P_{2y} subtype or UTP sensitive receptors and other purine or pyrimidine receptor agonists were generated, measuring mean change in [Ca²⁺]ᵢ in response to agonist concentration. Data are shown in **Figure 3**, which gives log concentration-effect relationships of purinergic and pyrimidinergic compounds on [Ca²⁺]ᵢ (mean change in [Ca²⁺]ᵢ over basal levels). **Fig. 3A** shows a comparison of agonists which bind P₂ₓ, P_{2y} or UTP sensitive receptors, mean basal [Ca²⁺]ᵢ in each set of experiments were (in nM): UTP (○), 81±8, range 48-113, n=5 (n=3-6); ATPγS (●), 116±11, range 85-151, n=4 (n=3-6); ATP (□), 61±3, range 51-70, n=8 (n=3-8 at each agonist concentration); 2MeSATP (■), 123±19 range 95-180, n=3 (n=3); ADPβS (Δ), 91±17, range 74-107, n=3 (n=3); αβMeATP (◇), 82±16, range 50-99, n=3 (n=3); βγMeATP (▲), 68±4, range 61-74, n=3 (n=3). Figure 3B shows a comparison of other purine agonists with response stimulated by ATP (○), mean basal [Ca²⁺]ᵢ in each set of experiments were (nM) : GTP (•), 117±13, range 92-153, n=3 (n=3); AMPPNP (□), 137±50, range 74-235, n=3 (n=3); ITP (■), 103±10, range 85-121, n=3 (n=3); ADP (Δ), 94±16, range 75-127, n=3 (n=2-3); AMP (▲), 111±28, range 69-189, n=3 (n=3); ATPαS (o), 89±2, range 87-91, n=1 (n=2). **Figure 3C** shows a comparison of UTP (○) with other pyrimidine agonists, mean basal [Ca²⁺]ᵢ (in nM): 5BrUTP (●), 110±13, range 82-152, n=3 (n=2-3); UDP (□), 89±2, range 84-92, n=3 (n=3); CTP (Δ), 83±10, range 69-102, n=3 (n=3); UMP (■), 78±14, range 64-91, n=2 (n=2).

ATP, UTP and ATPγS were the most effective agonists. Classical P₂ₓ (αβMeATP and βγMeATP) and P_{2y} (2MeSATP and ADPβS) receptor agonists had little effect (Figure 3A) as did other analogs of ATP and ADP (Figure 3B). 5BrUTP was essentially as effective as UTP for stimulation of Ca²⁺ mobilization (Figure 3C).

### EXAMPLE 6

### Cystic Fibrosis Nasal Epithelium

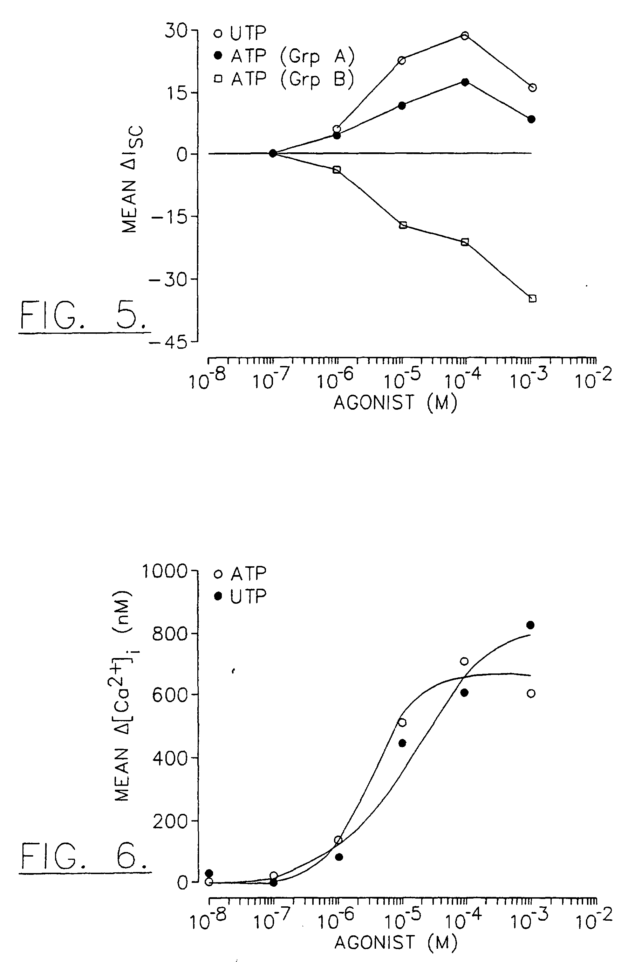

Availability of Cystic Fibrosis (CF) tissues is limited, and our investigation of effects of nucleotides was restricted to examining regulation of Cl⁻ secretion rates and [Ca²⁺]ᵢ levels by ATP and UTP. Only small changes in Cl⁻ secretion (amiloride-resistant I_{sc}) were observed in CF tissues following basolateral addition of ATP [14±3 maximum mean % change in I_{sc} (n=6)] compared with normal tissues [51±8 maximum mean % change in I_{sc} (n=6)]. Apical administration of ATP following blockade of Na+ absorption with amiloride resulted in two distinct patterns of response in tissues from CF subjects. Data are given in **Figures 4 and 5**.

Figure 4 provides representative bioelectric tracings of effect on I_{sc} of extracellular ATP (10⁻⁴ M) or UTP (10⁻⁴ M) applied to the apical surface of amiloride-pretreated (10⁻⁴ M) CF human nasal epithelium. **(A)** Cl⁻ secretion in response to ATP (post-amiloride I_{sc}=13 µA.cm⁻²). **(B)** I_{sc} response to ATP of opposite polarity (post-amiloride I_{sc}=19 µA.cm⁻²) **(C**) Cl⁻ secretory response to UTP (post-amiloride I_{sc}=5 µA.cm⁻²).

Figure 5 shows log concentration-effect curves for changes in I_{sc} from basal levels when ATP or UTP are applied to the apical surface of amiloride-pretreated (10⁻⁴ M) CF tissues. Mean post-amiloride I_{sc} in each set of experiments were (in µA.cm⁻²) : ATP [Grp A (●), Grp B (□)], 13±1, range 3-31, n=4 (n=4-13 at each agonist concentration); UTP (○), 12±2, range 8-14, n=3 (n=3). s.e. of each data point is ≤ 13 % of the normalized maximum response.

Most tissues exhibited an increase in I_{sc} over basal levels after ATP (Figure 4A), suggesting stimulation of Cl⁻ secretion. However, approximately 30% of CF tissues tested responded with a change to opposite polarity of I_{sc} following ATP (Figure 4B), suggesting a secretion dominated by cations in these preparations (Bean and D. Friel, *In: Ion Channels,* **Vol. 2**, 169-203 (T. Narahashi, Ed. 1990)). UTP applied to the apical surface routinely stimulated increases in Cl⁻ secretion in CF tissues (Figure 4C). Mean peak change in I_{sc} from post-amiloride basal levels in response to ATP or UTP application was measured in individual CF tissues to obtain the concentration-effect relationships illustrated in Figure 5.

Increases in [Ca²⁺]ᵢ in CF tissues were also observed after addition of ATP or UTP. Data are given in **Figure 6** which shows the log concentration-effect relationships of the effect of ATP and UTP on [Ca²⁺]ᵢ (change from basal levels) in single CF nasal epithelial cells. Mean basal [Ca²⁺]ᵢ (in nM): ATP (○), 65±6, range 44-86, n=3 (n=3 at each agonist concentration); UTP (●), 67±3, range 56-79, n=3 (n=3-4). s.e. of each data point is ≤ 8 % of the normalized maximum response. The similar potency and effectiveness of ATP and UTP in these tissues suggests the presence of a P₂ receptor type sensitive to UTP on CF epithelium.

## Claims

1. A pharmaceutical formulation comprising a compound of Formula I below, or a pharmaceutically acceptable salt thereof, in an amount effective to hydrate lung mucous secretions, in a pharmaceutically acceptable carrier: 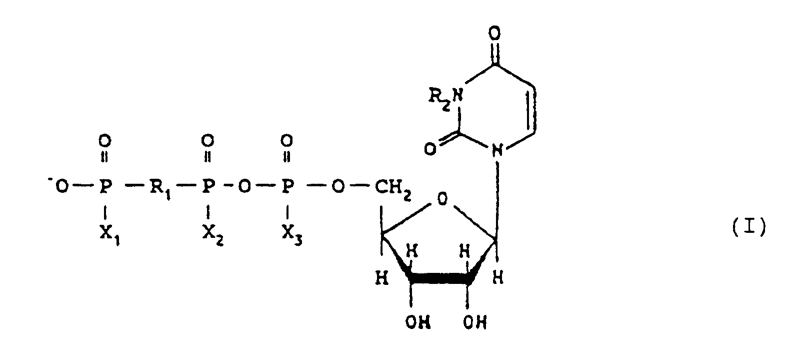 wherein:
X₁ is S⁻;
X₂ and X₃ are each independently selected from the group consisting of O⁻ and S⁻;
R₁ is selected from the group consisting of O, imido, methylene, and dihalomethylene; and
R₂ is H.

2. A pharmaceutical formulation according to claim 1, further comprising amiloride in an amount effective to inhibit the reabsorption of water from lung mucous secretions.

3. A pharmaceutical formulation according to claim 1, wherein said carrier is selected from the group consisting of solid carriers and liquid carriers.

4. A pharmaceutical formulation according to claim 1, wherein X₂ and X₃ are O⁻.

5. A pharmaceutical formulation according to claim 1, wherein R₁ is oxygen.

6. A pharmaceutical formulation according to claim 1, wherein said compound is uridine 5'-O-(3-thiotriphosphate), and the pharmaceutically acceptable salts thereof.

7. A pharmaceutical formulation comprising a compound of Formula I below, or a pharmaceutically acceptable salt thereof, in an amount effective to hydrate lung mucous secretions, in a pharmaceutically acceptable carrier: 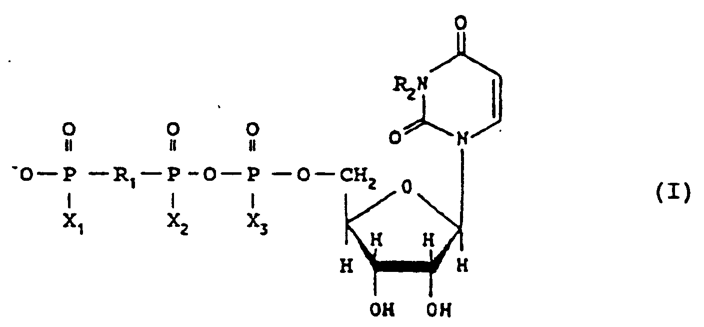 wherein:
X₁, X₂, and X₃ are each independently selected from the group consisting of O⁻ and S⁻;
R₁ is selected from the group consisting of O, imido, methylene, and dihalomethylene; and
R₂ is H;
said compound of Formula I subject to the proviso that uridine 5'-triphosphate is excluded therefrom.

8. A pharmaceutical formulation according to claim 7, wherein X₂ and X₃ are O⁻.

9. A pharmaceutical formulation according to claim 7, wherein R₁ is oxygen.

10. A pharmaceutical formulation comprising amiloride in an amount effective to inhibit the reabsorption of water from lung mucus secretions and a compound of Formula I below, or a pharmaceutically acceptable salt thereof, in an aerosolizable form to be delivered to the lungs of a subject in an amount effective to hydrate lung mucous secretions, in a pharmaceutically acceptable carrier: 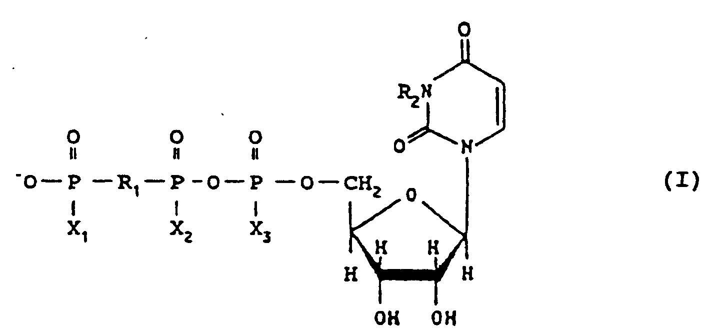 wherein:
X₁, X₂, and X₃ are each independently selected from the group consisting of O⁻ and S⁻;
R₁ is selected from the group consisting of O, imido, methylene, and dihalomethylene; and
R₂ is H;
said compound of Formula I subject to the proviso that uridine 5'-triphosphate is excluded therefrom.

11. A pharmaceutical formulation according to claim 10, wherein said carrier is selected from the group consisting of solid carriers and liquid carriers.

12. A pharmaceutical formulation according to claim 10, wherein X₂, and X₃ are O⁻.

13. A pharmaceutical formulation according to claim 10, wherein R₁ is oxygen.

14. A composition according to claim 10, wherein said compound of Formula I is in the form of respirable particles ranging in size from 1 to 10 microns.

## Patentansprüche

1. Pharmazeutische Zubereitung, umfassend eine Verbindung der nachstehenden Formel I oder ein pharmazeutisch verträgliches Salz davon in einer Menge, die wirksam Lungenschleimabsonderungen hydratisiert, in einem pharmazeutisch verträglichen Träger 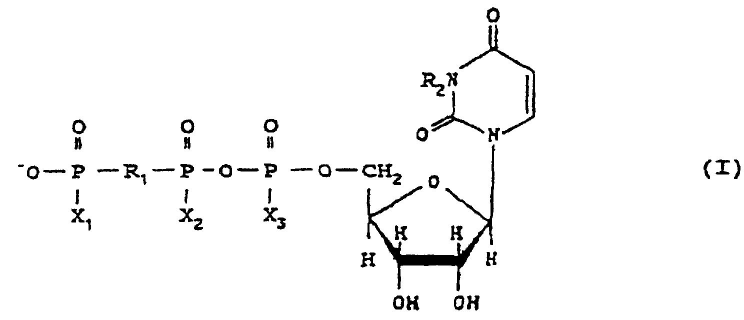 wobei:
X₁ S⁻ ist;
X₂ und X₃ jeweils unabhängig aus der Gruppe, bestehend aus O⁻ und S⁻, ausgewählt sind;
R₁ aus der Gruppe, bestehend aus O, Imido, Methylen und Dihalogenmethylen, ausgewählt ist; und
R₂ H ist.

2. Pharmazeutische Zubereitung nach Anspruch 1, weiterhin umfassend Amilorid in einer Menge, die wirksam die Resorption von Wasser aus Lungenschleimabsonderungen verhindert.

3. Pharmazeutische Zubereitung nach Anspruch 1, wobei der Träger aus der Gruppe, bestehend aus festen Trägern und flüssigen Trägern, ausgewählt ist.

4. Pharmazeutische Zubereitung nach Anspruch 1, wobei X₂ und X₃ O⁻ sind.

5. Pharmazeutische Zubereitung nach Anspruch 1, wobei R₁ Sauerstoff ist.

6. Pharmazeutische Zubereitung nach Anspruch 1, wobei die Verbindung Uridin-5'-O-(3-thiotriphosphat) und die pharmazeutisch verträglichen Salze davon sind.

7. Pharmazeutische Zubereitung, umfassend eine Verbindung der nachstehenden Formel I oder ein pharmazeutisch verträgliches Salz davon in einer Menge, die wirksam Lungenschleimabsonderungen hydratisiert, in einem pharmazeutisch verträglichen Träger: 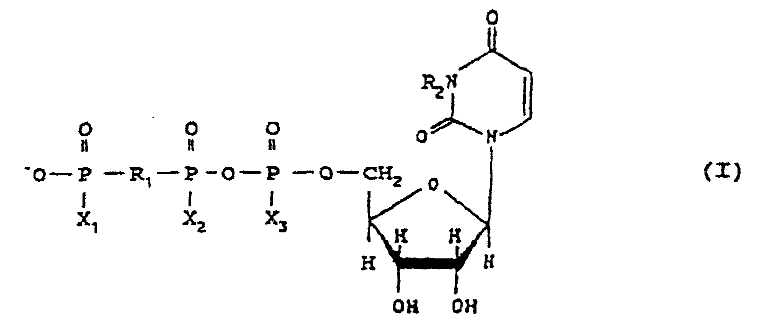 wobei:
X₁, X₂ und X₃ jeweils unabhängig aus der Gruppe, bestehend aus O⁻ und S⁻, ausgewählt sind;
R₁ aus der Gruppe, bestehend aus O, Imido, Methylen und Dihalogenmethylen, ausgewählt ist; und
R₂ H ist;
wobei die Verbindung der Formel I dem Vorbehalt unterworfen ist, daß Uridin-5'triphosphat davon ausgeschlossen ist.

8. Pharmazeutische Zubereitung nach Anspruch 7, wobei X₂ und X₃ O⁻ sind.

9. Pharmazeutische Zubereitung nach Anspruch 7, wobei R₁ Sauerstoff ist.

10. Pharmazeutische Zubereitung, umfassend Amilorid in einer Menge, die wirksam die Resorption von Wasser aus Lungenschleimabsonderungen verhindert, und eine Verbindung der nachstehenden Formel I oder ein pharmazeutisch verträgliches Salz davon in einer vernebelbaren Form, um in die Lungen eines Patienten in einer Menge, die wirksam Lungenschleimabsonderungen hydratisiert, abgegeben zu werden, in einem pharmazeutisch verträglichen Träger: 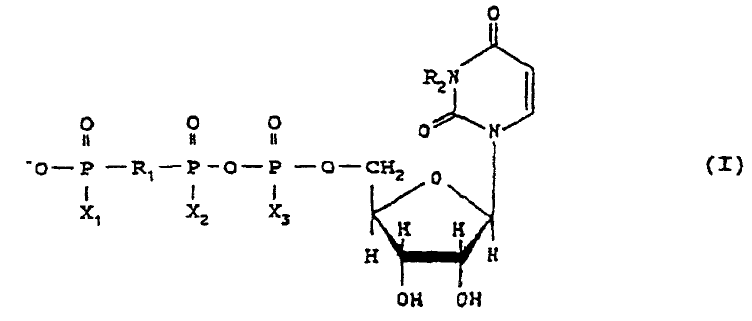 wobei:
X₁, X₂ und X₃ jeweils unabhängig aus der Gruppe, bestehend aus O⁻ und S⁻, ausgewählt sind;
R₁ aus der Gruppe, bestehend aus O, Imido, Methylen und Dihalogenmethylen, ausgewählt ist; und
R₂ H ist;
wobei die Verbindung der Formel I dem Vorbehalt unterworfen ist, daß Uridin-5'-triphosphat davon ausgeschlossen ist.

11. Pharmazeutische Zubereitung nach Anspruch 10, wobei der Träger aus der Gruppe, bestehend aus festen Trägern und flüssigen Trägern, ausgewählt ist.

12. Pharmazeutische Zubereitung nach Anspruch 10, wobei X₂ und X₃ O⁻ sind.

13. Pharmazeutische Zubereitung nach Anspruch 10, wobei R₁ Sauerstoff ist.

14. Zusammensetzung nach Anspruch 10, wobei die Verbindung der Formel I in der Form von zum Einatmen geeigneten Teilchen im Größenbereich von 1 bis 10 Mikrometern vorliegt.

## Revendications

1. Formulation pharmaceutique comprenant un composé de formule I ci-après, ou un sel pharmaceutiquement acceptable de celui-ci, en une quantité efficace pour hydrater des sécrétions muqueuses pulmonaires, dans un véhicule pharmaceutiquement acceptable: 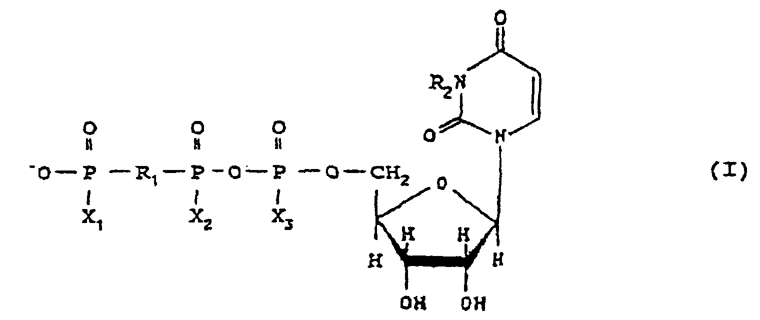 dans laquelle:
X₁ est S⁻;
X₂ et X₃ sont chacun indépendamment choisis dans le groupe constitué par O⁻ et S⁻;
R₁ est choisi dans le groupe constitué par O, un groupe imido, méthylène et dihalogénométhylène; et
R₂ est H.

2. Formulation pharmaceutique selon la revendication 1, comprenant en outre de l'amiloride en une quantité efficace pour inhiber la réabsorption d'eau à partir de sécrétions muqueuses pulmonaires.

3. Formulation pharmaceutique selon la revendication 1, dans laquelle ledit véhicule est choisi dans le groupe constitué par les véhicules solides et les véhicules liquides.

4. Formulation pharmaceutique selon la revendication 1, dans laquelle X₂ et X₃ sont O⁻.

5. Formulation pharmaceutique selon la revendication 1, dans laquelle R₁ est de l'oxygène.

6. Formulation pharmaceutique selon la revendication 1, dans laquelle ledit composé est le 5'-O-(3-thiotriphosphate) d'uridine, et les sels pharmaceutiquement acceptables de celui-ci.

7. Formulation pharmaceutique comprenant un composé de formule I ci-après, ou un sel pharmaceutiquement acceptable de celui-ci, en une quantité efficace pour hydrater des sécrétions muqueuses pulmonaires, dans un véhicule pharmaceutiquement acceptable: 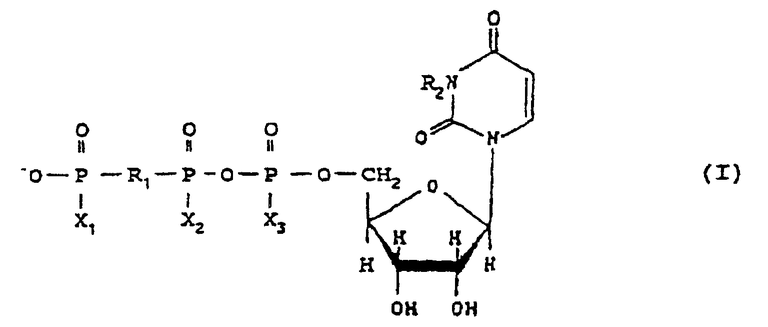 dans laquelle:
X₁, X₂ et X₃ sont chacun indépendamment choisis dans le groupe constitué par O⁻ et S⁻;
R₁ est choisi dans le groupe constitué par O, un groupe imido, méthylène et dihalogénométhylène; et
R₂ est H;
ledit composé de formule I étant soumis à la condition que le 5'-triphosphate d'uridine est exclu de celui-ci.

8. Formulation pharmaceutique selon la revendication 7, dans laquelle X₂ et X₃ sont O⁻.

9. Formulation pharmaceutique selon la revendication 7, dans laquelle R₁ est de l'oxygène.

10. Formulation pharmaceutique comprenant de l'amiloride en une quantité efficace pour inhiber la réabsorption d'eau à partir de sécrétions muqueuses pulmonaires et un composé de formule I ci-après, ou un sel pharmaceutiquement acceptable de celui-ci, sous une forme pouvant être mise en aérosol pour être administré aux poumons d'un patient en une quantité efficace pour hydrater des sécrétions muqueuses pulmonaires, dans un véhicule pharmaceutiquement acceptable: 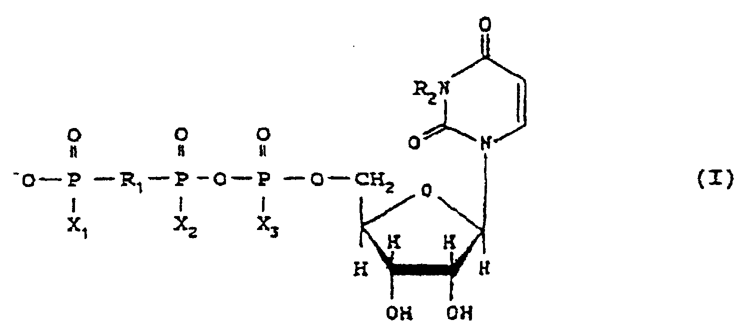 dans laquelle:
X₁, X₂ et X₃ sont chacun indépendamment choisis dans le groupe constitué par O⁻ et S⁻;
R₁ est choisi dans le groupe constitué par O, un groupe imido, méthylène et dihalogénométhylène; et
R₂ est H;
ledit composé de formule I étant soumis à la condition que le 5'-triphosphate d'uridine est exclu de celui-ci.

11. Formulation pharmaceutique selon la revendication 10, dans laquelle ledit véhicule est choisi dans le groupe constitué par les véhicules solides et les véhicules liquides.

12. Formulation pharmaceutique selon la revendication 10, dans laquelle X₂ et X₃ sont O⁻.

13. Formulation pharmaceutique selon la revendication 10, dans laquelle R₁ est de l'oxygène.

14. Composition selon la revendication 10, dans laquelle ledit composé de formule I est sous la forme de particules respirables allant en taille de 1 à 10 µm.
